# EUROPEAN PATENT SPECIFICATION

(11) **EP 1 430 859 B1**
(45) Date of publication and mention of the grant of the patent: **02.11.2006**
(21) Application number: 03257810.6
(22) Date of filing: 12.12.2003
(51) Int. Cl.: A61F 2/46, A61B 5/107, G01B 3/00

(54) **Adjustable biomechanical measuring instrument**
Verstellbare biomechanische Messvorrichtung
Instrument de mesure biomecanique réglable

(30) Priority: 20.12.2002 US 327187
(43) Date of publication of application: 23.06.2004
(73) Proprietor: DePuy Products, Inc., Warsaw, Indiana 46581 (US)
(72) Inventor: Dwyer, Kimberly A., FT Wayne Indiana (US); Rusbarsky, Christine, Clinton Connecticut (US)
(74) Representative: Belcher, Simon James

(56) References cited:
- EP-A- 0 381 893
- WO-A-02/17826
- FR-A- 2 416 002
- FR-A- 2 492 249
- FR-A- 2 684 287
- US-A- 3 271 868
- US-A- 4 131 998
- US-A- 4 658 808
- US-A- 5 792 143
- US-A- 6 027 507

## Description

The present invention relates to a tool for aiding in the selection of an appropriate sized implant for use in hip arthroplasty.

Document US-A-4 658 808 is considered to represent the closest prior art and discloses an arrangement of prosthesis templates comprising head/neck portions fixed on a template sheet, whereas the partial templates for the stems are fastened to plastic rails which in turn are displaceable in corresponding guides on the template sheet.

Patients who suffer from the pain and immobility caused by osteoarthritis and rheumatoid arthritis have an option of joint replacement surgery. Joint replacement surgery is quite common and it enables many individuals to function properly when it would not be possible otherwise to do so. Artificial joints usually comprise of metal, ceramic and /or plastic components that are fixed to existing bone.

Such joint replacement surgery is otherwise known as total joint arthroplasty. Total joint arthroplasty is a well-know surgical procedure by which a diseased and/or damaged joint is replaced with a prosthetic joint. In a typical total joint arthroplasty, the ends or distal portions of the bones adjacent to joint are resected, or a portion of the distal part of the bone is removed and the artificial joint is secured thereto.

Many designs and methods for manufacturing implantable articles, such as bone prostheses are known to exist. Such bone prostheses include components of artificial joints, such as elbows, hips, knees, and shoulders.

Currently, a major critical concern is the instability of the joint in total hip arthroplasty. Instability is associated with dislocation. Dislocation is particularly a problem in total hip arthroplasty.

Factors related to dislocation include surgical technique, implant design, implant positioning, and patient related factors. In total hip arthroplasty, implant systems address this concern by offering a series of products with a range of lateral offsets, neck lengths, head lengths, and leg lengths. The combination of these four factors affect the laxity of the soft tissue. By optimizing the biomechanics, the surgeon can provide the patient a stable hip much more resistant to dislocation.

In order to accommodate the range of patient and anthropometries, a wide range of hip implant geometries are currently manufactured by DePuy Orthopaedics, Inc. and by other companies. In particular, the total hip system offered by DePuy Orthopaedics, Inc. under the trade mark S-ROM includes four offsets, three neck lengths, four head lengths, and one leg length adjustment. The combination of all these biomechanical options is rather complex.

Currently, the surgeon utilizes a radiograph (x-ray) to assist in selecting the prosthesis for a patient with the proper lateral offsets, neck lengths, head lengths, and leg lengths. The surgeon utilizes the radiograph by overlaying a radiograph of the patient's femur and acetabulum with an acetate overlay. Each prosthesis implant corresponds to a particular acetate overlay. The surgeon picks the acetate overlay which most closely corresponds to the patient's natural femur. In addition, the surgeon may utilize a single nonadjustable instrument (for example DePuy instrument number 53-1420) during surgery to determine the head centre of the exposed bone. This single instrument or femur resection template only demonstrates one biomechanical combination and may not match what has been templated for the patient on the radiograph.

WO-02/17826 discloses a device for determining the optimal dimensions of the components of a knee endoprosthesis. The device comprises an angular element having a first anterior plate and a second distal plate that is arranged at right angles thereto. The device further comprises a first calliper. The first calliper is arranged on the second distal plate in a manner that permits it to be displaced parallel to the central axis and to be arrested. The first calliper has a bearing surface which is essentially parallel to the first plate and designed to rest against a tibial plateau when a knee joint is bent. The device also comprises a second calliper which is arranged in a manner that permits it to be displaced parallel to the longitudinal axis of the first plate and to be arrested. The second calliper also has a bearing surface which is essentially parallel to the inner side of the second plate and designed to rest against the tibial plateau when the knee joint is straightened.

A need therefore exists for a prosthetic implant with additional functionality.

The present invention includes an instrument. The instrument is an adjustable device that allows the user to slide the neck section in a medial lateral direction to template lateral offset. It may also slide in a proximal distal direction to the template leg length. The instrument may slide in a diagonal direction to template either the neck length or head length or a combination thereof. The actual lateral offset, leg length and neck length may be etched onto part of the instrument to indicate their amounts and to assist in implant selection.

The present invention provides an instrument for templating multiple biomechanical combinations with one instrument or tool. This templating may be achieved through multiple sliding mechanisms that allow the instrument to expand or contract to the desired lateral offset, neck length, head length, and/or leg length. The instrument may be utilized to template intraoperatively and preoperatively through the use of radiographs (x-rays).

According to one embodiment of the present invention, a tool for aiding in the selection of an appropriately sized implant for use in performing hip joint arthroplasty in cooperation with at least one of a hip joint and an image of a hip joint. The tool has a stem portion which can be aligned with the distal portion of the femur, a head portion which corresponds to the head of the femur which articulates with the acetabulum, and a neck portion on which the head portion is located, and thereby corresponding to the configuration of the femoral component of a hip joint prosthesis. The tool comprises:
a body having a proximal segment, in which the neck portion can be moved along the medial-lateral directions with respect to the proximal segment to enable lateral offset to be templated,
a head portion which is mounted on the neck portion, which can be moved in a diagonal direction with respect to the medial-lateral and proximal-distal axes with respect to the body to enable neck length or head length or both to be templated, and
a stem portion which extends from the body for alignment with the distal portion of the femur,
in which the movements of the neck portion and the head portion enable the relative positions of the head and body portions of the hip joint to be measured for use in the selection of an appropriately sized implant.

The technical advantages of the present invention include the ability of the instrument to demonstrate a complete range of biomechanical options contemplated in total hip arthroplasty procedures. For example, according to one aspect of the present invention, the instrument may adjustably telescope in three directions to duplicate lateral offset, leg length, and neck length. Thus, the present invention provides for ranges of biomechanical options including those of lateral offset, leg length, and neck length.

Another technical advantage of the present invention is the ability of the present invention to allow the surgeon to visualize head centre and level of resection before making any cuts to the bone. For example, according to one aspect of the present invention, the template may be positioned over the radiograph or x-ray and the template may be adjusted to a position corresponding to the natural femur and acetabulum. Thus, the present invention provides for visualization of head centre and levels of resection prior to making any cuts to the bone. The visualization prior to cutting may be either with the use of the instrument in radiographs or for the instrument to be utilized visually intraoperatively after the bone has been exposed prior to its resection.

Embodiments of the invention will now be described by way of example with reference to the accompanying drawings, in which:
FIG. 1 is plan view of an adjustable template in accordance with a first embodiment of the present invention with lateral offset, leg length, and head centre adjustments;
FIG. 2 is an exploded plan view of the adjustable template of FIG. 1;
FIG. 3 is a side view of the adjustable template of FIG. 1;
FIG. 4 is a plan view of the body of the template of FIG. 1;
FIG. 5 is a plan view of a mirror image body to that of FIG. 4;
FIG. 6 is a plan view of the first segment of the template of FIG. 1;
FIG. 7 is a side view of the second segment of the template of FIG. 1;
FIG. 8 is a plan view of the second segment of FIG. 7;
FIG. 9 is a rear view of the second segment of FIG. 7;
FIG. 10 is a plan view of head of the template of FIG. 1;
FIG. 11 is an end view of the head of FIG. 10;
FIG. 12 is an exploded partial plan of another embodiment of an adjustable template in accordance with the present invention.
FIG. 13 is a cross-sectional view along the lines 19-19 of FIG. 12 in the directions of the arrows;
FIG. 14 is a plan view of a modular hip stem for use with the template of FIG. 1;
FIG. 15 is an exploded plan view of the modular hip stem of FIG. 14;
FIG. 16 is a plan view of various sizes of the proximal bodies of the hip stem of FIG. 14;
FIG. 17 is a plan view of various sizes of the distal stem of the hip stem of FIG. 14; and
FIG. 18 is a plan view of various sizes of the head stem of the hip stem of FIG. 14.

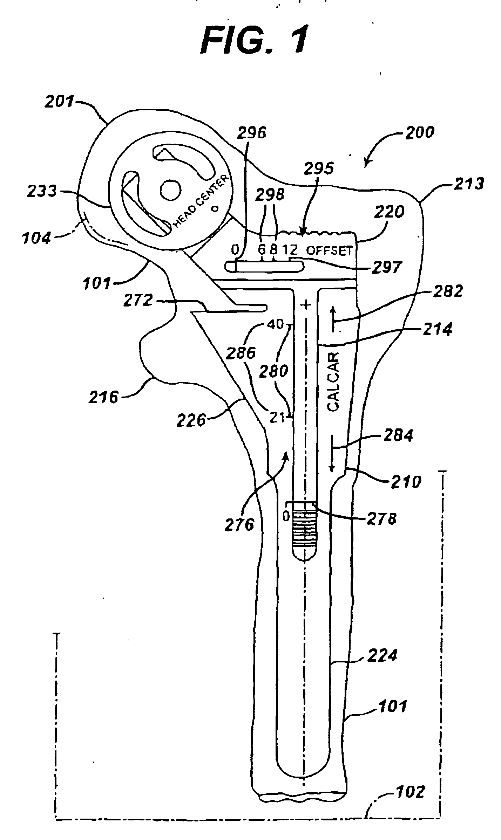

Referring to the drawing, FIG. 1 shows a tool 200 which can aid in the selection of an appropriately sized implant for use in hip joint arthroplasty. The tool 200 may be used in conjunction with an image of a joint from a radiograph or x-ray. The tool 200 as shown in FIG. 1 is used for arthroplasty of the left hip. The tool 200 may be made of any suitable, durable material and may be, for example, be made of a metal or a plastic. The tool 200 consists of a plurality of parts including a body 210. As shown in FIG. 1, the body 210 includes a stem portion 224 as well as a wider portion 226- The stem portion 224 is aligned with the distal portion of the femur 101, while the wider portion 226 is aligned with proximal femur.

The tool 200 further includes a proximal segment 214 which is slidably secured to the body 210. The proximal segment 214 is slidably movable within the body 210 in the direction of arrows 282 and 284. The tool 200 further includes a neck portion 220 which is slidably moveable with respect to the proximal segment 214. The tool 200 further includes a head portion 233 which is slidably moveable with respect to the neck portion 220. The head portion 233 is aligned with head 201 of the femur 101.

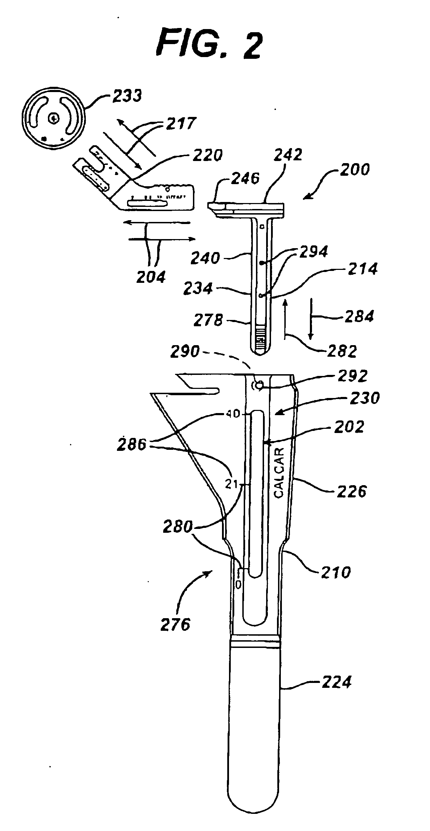

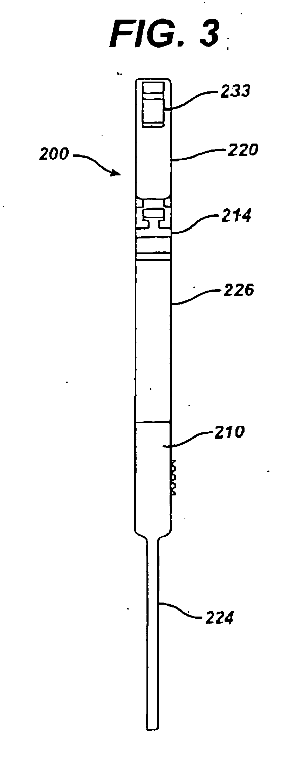

Referring now to FIGS. 2, 3 and 5, the body 210 is shown in greater detail. The body 210 includes the distal stem 224 and the wider portion 226. As shown in FIG. 2, the body 210 includes an opening 202 through the body 210. The opening 202 permits viewing of the proximal segment 214 as it moves up and down in the directions of arrows 282 and 284. The body 210 includes indicia 276 for assisting and determining the relative position of the body 210 with respect to the proximal segment 214.

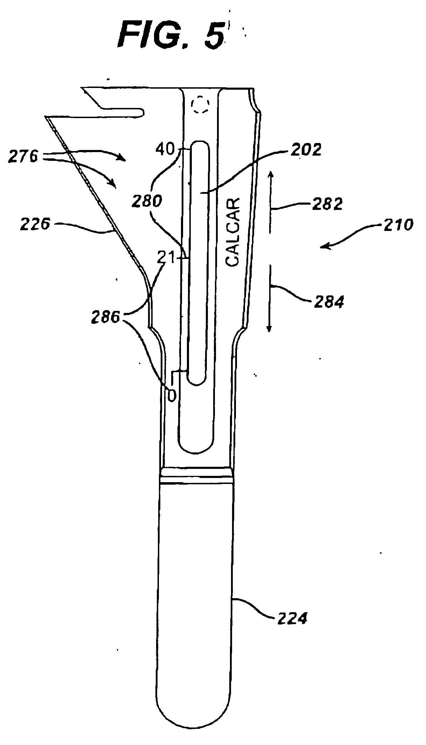

For example, as shown in FIG. 5, the indicia 276 includes a mark 278 located on the proximal segment 214 as well as marks in the form of lines 280 on the body 210- Each of the lines 280 may include numbers 286 thereby. The numbers 286 may be indicative of relative distance between the respective lines 280 or may correspond to the prosthesis that the tool 200 would indicate is appropriate.

Referring again to FIG. 2, the proximal segment 214 may be slidably engageable with the body 210 in the direction of arrows of 282 and 284 in any suitable way. For example, the body 210 may include a slot 230, while the proximal segment 214 may include a calcar slide 234. The slide 234 may slidably fit within the slot 230.

The proximal segment 214 may be selectively removably secured to the body 210 by, for example, a screw 292 which may be threadably engaged to the body 210 at hole 290 formed in the body 210. The screw 292 may include a spring biassed ball shaped contact point (not shown) which may contact recesses 294 formed in the proximal segment 214. The contact point of the screw 292 may be in contact with the recesses 294 as the proximal segment 214 is moved in the direction of 282 and 284 making the location of the positions indicated by the numbers 286 more easy to locate, especially when placed by an exposed femur.

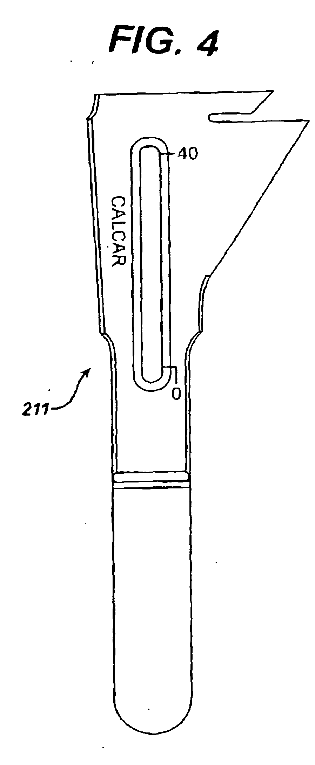

Referring now to FIG. 4, a body 211 is shown which is a mirror image of the body 210 and may be used with a right hip arthroplasty. It should be appreciated that mirror images of proximal segment 214, neck portion 220, and head portion 233 can be used with the body 211 to form a mirror image tool to that of tool 300.

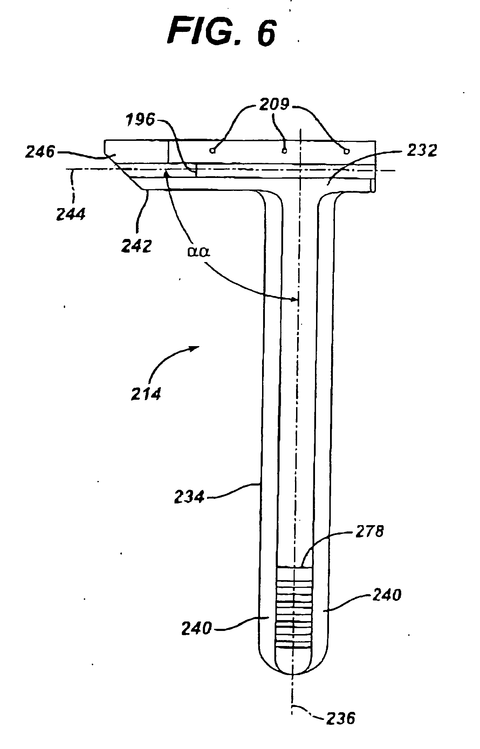

Referring now to FIG. 6, the proximal segment 214 is shown in greater detail. The proximal segment 214 includes a central base 232. Extending from the central base 232 along first centre line 236 is calcar slide 234. Also extending from the base 232 at an angle αα from the first centre line 236 is an offset slide 242 which extends along second centre line 244.

Referring again to FIG. 2, the calcar slide 234 slides in the directions of arrows 282 and 284 along slot 230. The calcar slide is guided and retained by rails 240 extending from the calcar slide 234. A mark 278 is located on the calcar slide to assist in determining the relative location of the proximal segment 214 with respect to the body 210. The offset slide 242 is slidably engaged with the neck portion 220 and is guided by rails 246 extending from the offset slide 242.

Referring again to FIG. 2, the neck portion 220 is slidably movable in the direction of arrows 204 along offset slide 242.

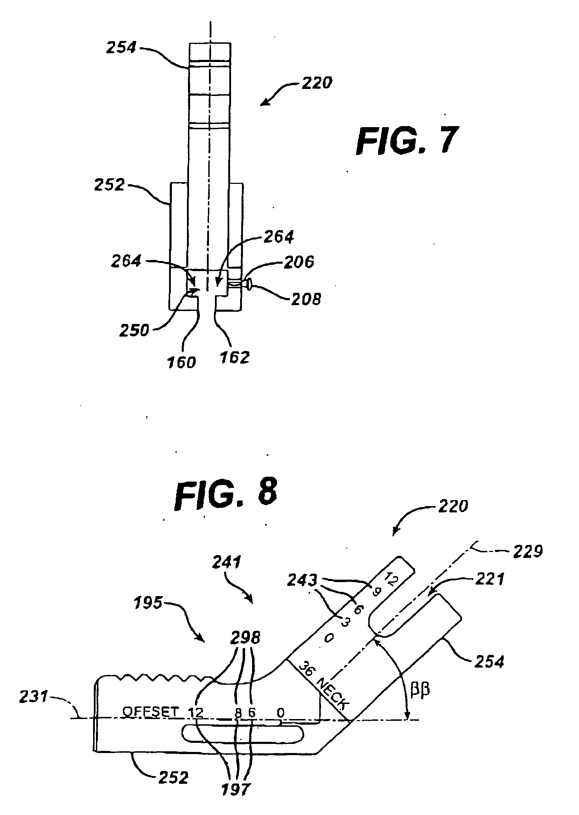

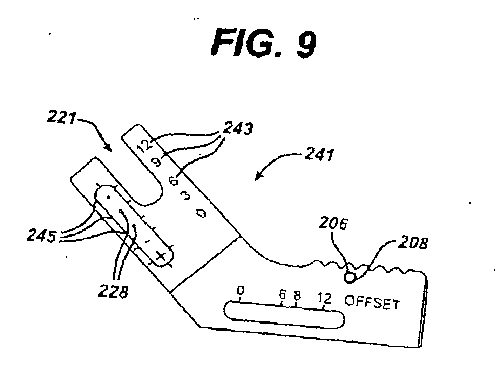

Referring now to FIGS. 7, 8, and 9, the neck portion 220 is shown in greater detail. The neck portion 220 includes a neck 254 and a slide portion 252. The slide portion 252 includes a slot 250 which cooperates with the offset slide 242 of the proximal segment 214. The slot 250 is defined by lips 260 and 262 which form grooves 264 which mate with rails 246 of the offset slide 242 (see FIG. 2).

Preferably, and as shown in FIGS. 6 and 8, the tool 200 includes indicia 295 for determining the relative position of the neck portion 220 with respect to the proximal segment 214. The indicia 195 may, for example, be in the form of a plurality of reference marks 197 on one of the proximal segment 214 and the neck portion 220 and a solitary mark on the other of the proximal segment 214 and the neck portion 220. For example, the neck portion 220 may include a plurality of reference marks 197 and the proximal segment 214 may include a reference mark 278. Reference numerals 298 may be positioned adjacent the reference marks 197 to assist in the relative positioning of the neck portion 220 with respect to the proximal segment 214. The reference numerals 298 may denote relative positions of the reference marks or may correspond to the particular size or number of orthopaedic implants corresponding to the proper implant that the tool 200 would suggest.

Referring to FIGS. 6 through 8, the neck portion 220 may be selectively secured to the proximal segment 214 in any suitable manner. For example, the neck portion 220 may include a locking feature for selectively fixedly securing the neck portion 220 to the proximal segment 214. For example, the slide portion 252 may include a threaded hole 206 which passes into the slot 250. A screw 208 may be threadably secured to the threaded hole 206. The screw 206 may be similar to the screw 292 used on the body 210. The screw 208 may include a spring-loaded ball tip. The offset slide 242 of the proximal segment 214 may include a plurality of indents 209 which provide for preset positions in alignment with the reference numerals 298.

Referring again to FIG. 2, the head portion 233 is slidably connected to the neck portion 220 and is permitted to move in the direction of arrows 217.

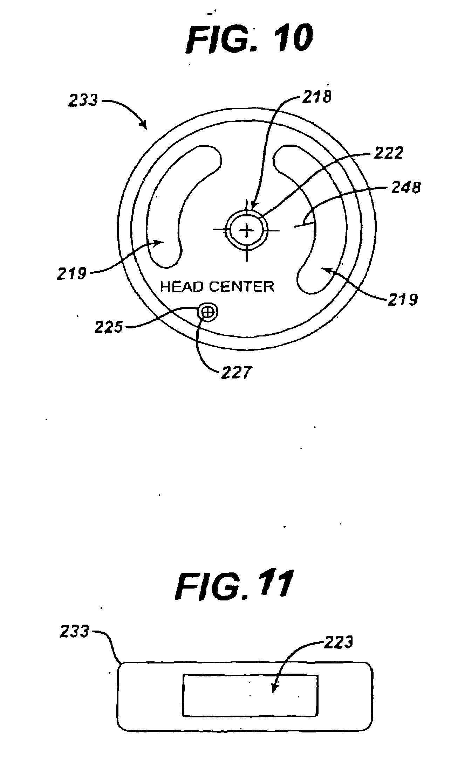

Referring now to FIGS. 8, 9, 10 and 11, the head portion 233 is shown in greater detail. The head portion 233 has a generally cylindrical shape and as shown in FIG. 10 includes a central hole 218 as well as two arcuate openings 219. The head portion 233 may be slidably secured to the neck 254 of the neck portion 220 in a suitable fashion. For example, a pin 219 may be fittably secured to the hole 218 in the head portion 233. The pin 219 may be slidably fitted to opening 221 in the neck 254 of the neck portion 220.

Alternatively, or in addition to the use of the pin 219, the head portion 233 may include a central slot 223 into which the neck 254 may be slidably fitted.

The head portion 233 may be selectably fixedly secured to the neck portion 220 in any suitable fashion. For example, the head portion 233 may include a threaded hole 225 to which screw 227 may be threadably attached. The screw 227 may be similar to the screw 208 and may include a spring biassed spherical tip. To provide for preset positions, the neck portion 220 may include a plurality of indents 228 which may be aligned with the screw 227 to provide for preselected positions. The preselected positions may be in alignment with reference numerals 243.

Referring to FIGS. 8 through 10, the neck 254 of the neck portion 220 may extend from the slide portion 252 of the neck portion 220 along a neck centre line 229 which extends at an angle ββ from the slide centre line 231 of the slide portion 252.

To assist in observing the position of the head portion 233 with respect to the neck portion 220, the neck portion 220 and/or the head portion 233 may include indicia 241 for determining the relative location of the head portion 233 with respect to the neck portion 220. For example, the indicia 241, may be in the form of a plurality of marks 245 on the neck 254 of the neck portion 220 and a solitary mark 248 located on the head portion 233. In addition, the indicia 241 may include reference numbers 243 which may be positioned in alignment with the marks 245 and which may correspond to particular spacing distance between the adjacent marks 245 or correspond to the suggested implant that corresponds with the particular settings determined on the tool 200.

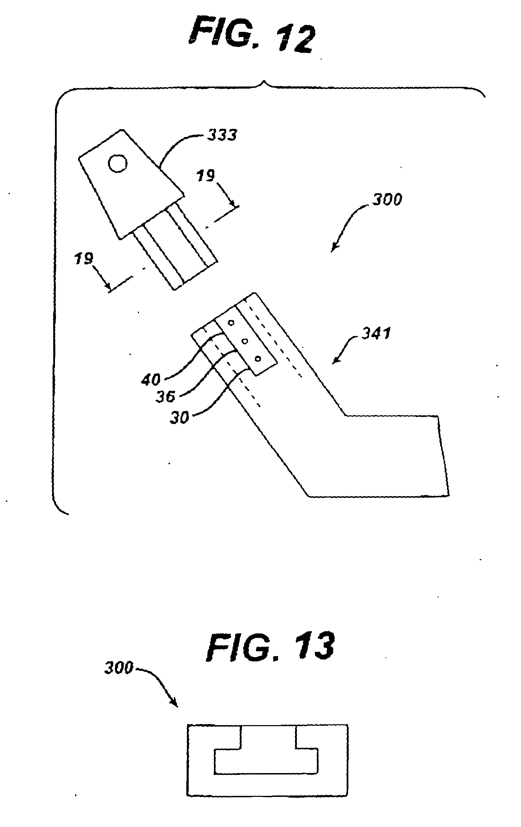

Referring now to FIGS. 12 and 13, an alternate embodiment of the present invention is shown as tool 300. Tool 300 is similar to the tool 200 of FIGS. 1 through 11 except that in place of the head portion 233 of the tool 200, the tool 300 includes a head portion 333 which has a generally tapered shape and to correspond with the tapered shape of a neck of a prosthetic stem. It should be appreciated that tool 300 may further include indicia 341 similar to the indicia 195 of the second segment 220 of the tool 200.

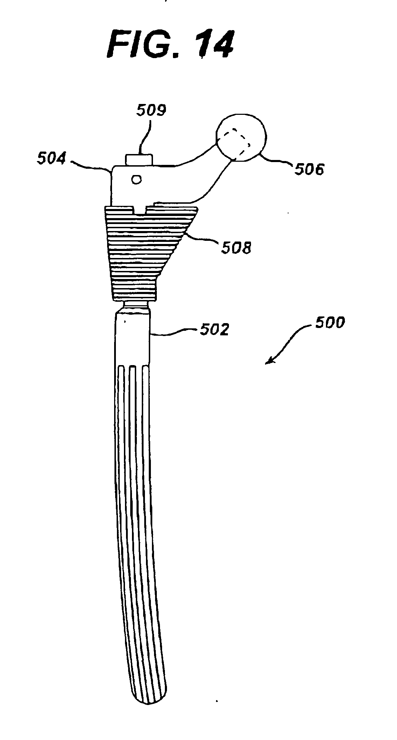

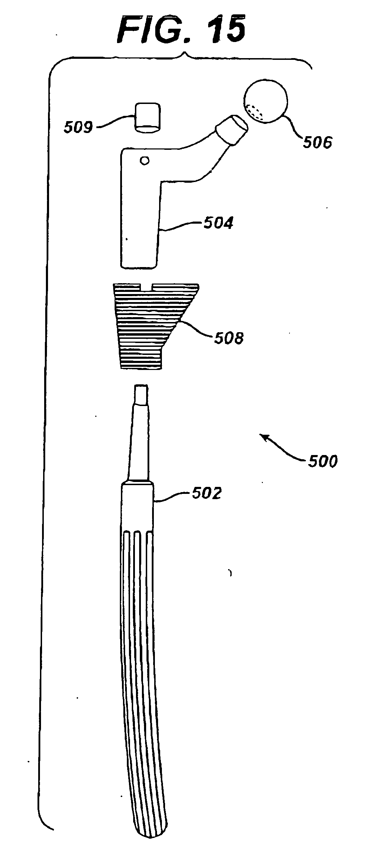

Referring now to FIGS. 14 and 15, a multi piece prosthetic hip femoral component 500 is shown. The femoral component 500 includes a distal stem 502 which is connected to a proximal body 504. A head 506 is connected to the proximal body 504. A sleeve 508 is fitted over the proximal body 504. A nut 509 is used to secure the proximal body to the distal stem 502.

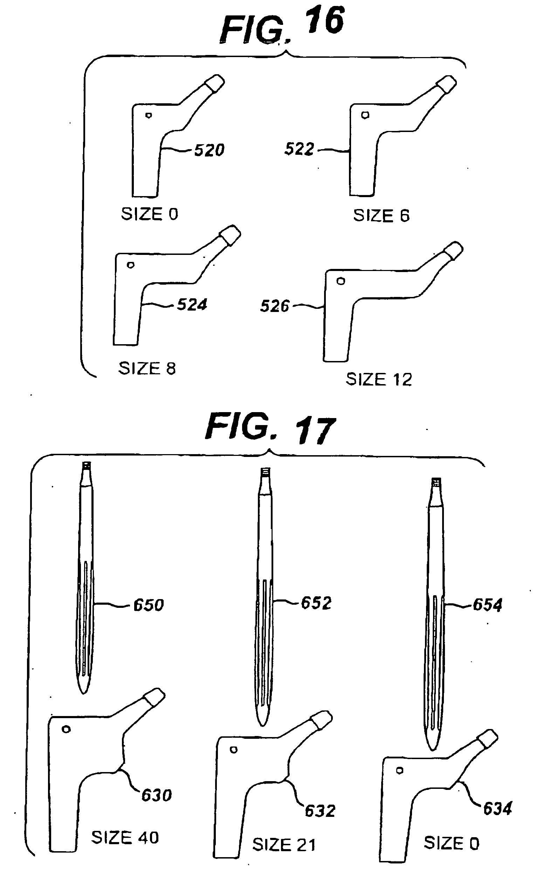

Referring now to FIGS. 16, 17 and 18, a plurality of distal stems and proximal bodies, and head5 are shown for use in conjunction with the tool 200 of FIG, 1 through 11. For example, in referring to FIGS. 1 and 16, the proximal body may be selected with the proper offset by doing the proper offset from the neck portion 220 of the tool 200 and observing the proper number 298. By referring to the proper number 298, the corresponding appropriate proximal body may be selected. For example, for offset 0, the proximal body 520 would be selected, for the offset size 6, the proximal body 522 would be selected, for the offset size 8, the proximal body 524 would be selected and for the offset size 12, the proximal body 526 would be selected.

Referring now to FIGS. 1 and 17, the appropriate proximal body stem would be selected by observing the calcar size recommendation from the tool 200 observed from the reference numeral 286 on the body. For example, for reference numeral 40, the proximal body 630 would be selected. For the calcar size 21, the proximal body 632 would be selected and for the calcar size 0, the proximal body 634 would be selected.

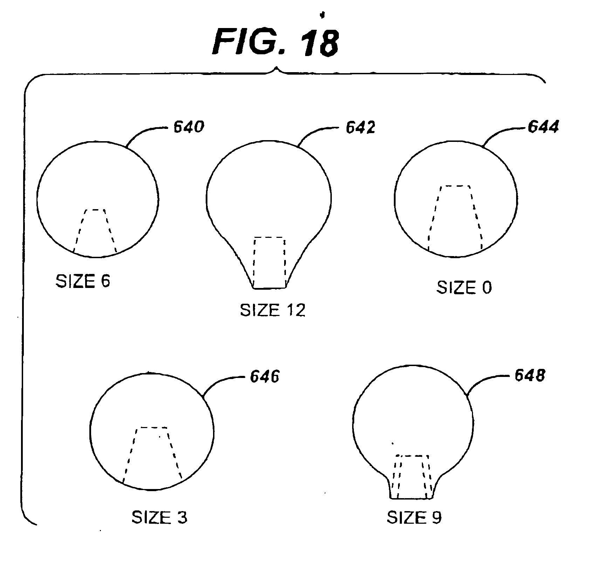

Referring now to FIG. 18 and FIG. 8, the appropriate head would be selected based on the reference numbers 243 selected from the indicia 241 as shown in FIG. 8. For example, for a head centre number 6, the head 640 would be selected, for the head centre 12, the head 642 would be selected, for the head centre 0, the head 644 would be selected, for the head centre 3, the head 646 would be selected, and for the head centre number 9, the head 648 would be selected.

## Claims

1. A tool (200) for aiding in the selection of an appropriately sized implant for use in performing hip joint arthroplasty in cooperation with at least one of a hip joint and an image of a hip joint, said tool (200) having a stem portion (224) which can be aligned with the distal portion of the femur (101), a head portion (233) which corresponds to the head (201) of the femur (101) which articulates with the acetabulum, and a neck portion (220) on which the head portion (233) is located, and thereby corresponding to the configuration of the femoral component of a hip joint prosthesis, and
a body (210) having a proximal segment (214), in which the neck portion (220) can be moved along the medial-lateral directions (204) with respect to the proximal segment (214) to enable lateral offset to be templated, wherein
the head portion (233) is mounted on the neck portion (220) and can be moved in a diagonal direction (217) with respect to the medial-lateral (204) and proximal-distal (282 and 284) axes with respect to the body (210) to enable neck length or head length or both to be templated, and
the stem portion (224) extends from the body (210) for alignment with the distal portion of the femur (101),
the movements of the neck portion (220) and the head portion (233) enabling the relative positions of the head and body portions of the hip joint to be measured for use in the selection of an appropriately sized implant.

2. The tool (200) of claim 1, in which the body (210) has a channel (230) formed therein and the proximal segment (214) can slide within the channel (230).

3. The tool (200) of claim 1, in which at least one of the proximal segment (214) and the neck portion (220) has indicia (295) thereon for indicating the relative positions of the neck portion (220) with respect to the proximal segment (214).

4. The tool (200) of claim 1, in which at least one of the body (210) and the proximal segment (214) has a portion which is translucent.

5. The tool (200) of claim 1, in which the proximal segment (214) can be moved along the proximal-distal direction (282 and 284) with respect to the body (210) to enable leg length to be templated.

6. The tool of claim 1, in which at least one of the body (210) and the proximal segment (214) has indicia (276) thereon for indicating the relative positions of the body (210) with respect to the proximal segment (214).

## Patentansprüche

1. Werkzeug (200) zur Unterstützung bei der Auswahl eines geeignet großen Implantats zur Verwendung bei der Durchführung einer Hüftgelenkplastik, zusammen mit einem Hüftgelenk und/oder einem Bild eines Hüftgelenks, wobei das Werkzeug (200) umfasst: einen Schaftabschnitt (224), der an dem distalen Abschnitt des Femurs (101) ausgerichtet werden kann, einem Kopfabschnitt (233), der dem Kopf (201) des Femurs (101) entspricht, der mit der Hüftgelenkpfanne ein Gelenk bildet, und einen Halsabschnitt (220), auf dem der Kopfabschnitt (233) angeordnet ist, wodurch dieses dem Aufbau der Femurkomponente einer Hüftgelenkprothese entspricht, und
einen Körper (210) mit einem proximalen Abschnitt (214), in dem der Halsabschnitt (220) entlang der medialen-lateralen-Richtungen (240) relativ zu dem proximalen Abschnitt (214) bewegt werden kann, um das Einstellen eines lateralen Versatzes zu ermöglichen, wobei
der Kopfabschnitt (231) auf dem Halsabschnitt (220) angeordnet ist und in einer diagonalen Richtung (217) relativ zu der medialen-lateralen-Achse (204) und der proximalen-distalen-Achse (282 und 284) bezogen auf den Körper (210) bewegt werden kann, um das Einstellen der Halslänge und/oder der Kopflänge zu ermöglichen, und wobei
der Schaftabschnitt (224) sich von dem Körper (210) erstreckt, für eine Ausrichtung an dem distalen Abschnitt des Femurs (101),
die Bewegungen des Halsabschnitts (220) und des Kopfabschnitts (233) ein Messen der relativen Lagen des Kopf- und Körperabschnitts des Hüftgelenks ermöglichen, zur Verwendung bei der Auswahl eines geeignet großen Implantats.

2. Werkzeug (200) nach Anspruch 1, **dadurch gekennzeichnet, daß** der Körper (210) einen Kanal (230) aufweist, der darin gebildet ist, und daß der proximale Abschnitt (214) in dem Kanal (230) gleiten kann.

3. Werkzeug (200) nach Anspruch 1, **dadurch gekennzeichnet, daß** der proximale Abschnitt (214) und/oder der Halsabschnitt (220) eine Anzeige (295) darauf aufweisen, um die relativen Lagen des Halsabschnitts (220) relativ zu dem proximalen Abschnitt (214) anzuzeigen.

4. Werkzeug (200) nach Anspruch 1, **dadurch gekennzeichnet, daß** der Körper (210) und/oder das proximale Segment (214) einen lichtdurchlässigen Abschnitt aufweisen.

5. Werkzeug (200) nach Anspruch 1, **dadurch gekennzeichnet, daß** der proximale Abschnitt (214) entlang der proximalen-distalen-Richtung (282 und 284) relativ zu dem Körper (210) bewegt werden kann, um das Einstellen der Beinlänge zu ermöglichen.

6. Werkzeug nach Anspruch 1, **dadurch gekennzeichnet, daß** der Körper (210) und/oder der proximale Abschnitt (214) eine Anzeige (276) darauf aufweisen, um die relativen Lagen des Körpers (210) im Vergleich zu dem proximalen Abschnitt (214) anzuzeigen.

## Revendications

1. Outil (200) pour faciliter le choix d'un implant de taille appropriée destiné à être utilisé pour effectuer une arthroplastie de la hanche en coopération avec au moins un parmi une articulation de la hanche et une image d'une articulation de la hanche, ledit outil (200) comportant une partie de tige (224) pouvant être alignée avec la partie distale du fémur (101), une partie de tête (233) qui correspond à la tête (201) du fémur (101) qui s'articule avec l'acétabulum et une partie de cou (220) sur laquelle est disposée la partie de tête (233) et correspondant ainsi à la configuration du constituant fémoral d'une prothèse de hanche, et
un corps (210) comportant un segment proximal (214), dans lequel on peut déplacer la partie de cou (220) dans les directions médiane-latérale (204) par rapport au segment proximal (214) pour permettre de modéliser le décalage latéral, dans lequel
la partie de tête (233) est montée sur la partie de cou (220) et on peut la déplacer dans une direction diagonale (217) par rapport aux axes médian-latéral (204) et proximal-distal (282 et 284) par rapport au corps (210) pour permettre de modéliser la longueur de cou ou la longueur de tête ou les deux, et
la partie de tige (224) s'étend depuis le corps (210) pour alignement avec la partie distale du fémur (101),
les mouvements de la partie de cou (220) et de la partie de tête (233) permettant de mesurer les positions relatives des parties de tête et de corps de l'articulation de la hanche pour être utilisées dans le choix d'un implant de taille appropriée.

2. Outil (200) selon la revendication 1, dans lequel le corps (210) comporte un canal (230) formé à l'intérieur et le segment proximal (214) peut coulisser dans le canal (230).

3. Outil (200) selon la revendication 1, dans lequel au moins un parmi le segment proximal (214) et la partie de cou (220) comporte une indication (295) sur celui-ci pour indiquer les positions relatives de la partie de cou (220) par rapport au segment proximal (214).

4. Outil (200) selon la revendication 1, dans lequel au moins un parmi le corps (210) et le segment proximal (214) comporte une partie qui est transparente.

5. Outil (200) selon la revendication 1, dans lequel le segment proximal (214) peut être déplacé dans la direction proximale-distale (282 et 284) par rapport au corps (210) pour permettre de modéliser la longueur de la jambe.

6. Outil selon la revendication 1, dans lequel au moins un parmi le corps (210) et le segment proximal (214) comporte une indication (276) sur celui-ci pour indiquer les positions relatives du corps (210) par rapport au segment proximal (214).
